# FASCICULE DE BREVET EUROPEEN

(11) **EP 1 131 111 B1**
(45) Date de publication et mention de la délivrance du brevet: **26.03.2003**
(21) Numéro de dépôt: 99972114.5
(22) Date de dépôt: 18.11.1999
(51) Int. Cl.: A61L 27/00, A61F 2/12

(54) **IMPLANT INJECTABLE COMPRENANT DES MICROPARTICULES POREUSES**
PORÖSE MIKROPARTIKEL ENTHALTENDE INJIZIERBARE IMPLANTATIONSZUSAMMENSETZUNG
INJECTION IMPLANT COMPRISING POROUS MICROPARTICLES

(30) Priorité: 18.11.1998 FR 9814679
(43) Date de publication de la demande: 12.09.2001
(73) Titulaire: Procytech, 33650 Martillac (FR)
(72) Inventeur: BISSON, Jean-Louis, F-33000 Bordeaux (FR)
(74) Mandataire: Ulmann, Catherine Claire
(86) Numéro de dépôt international: FR9902824
(87) Numéro de publication internationale: WO00029042

(56) Documents cités:
- EP-A- 0 073 593
- EP-A- 0 730 847
- WO-A-93/15721
- US-A- 5 336 263
- DATABASE WPI Section Ch, Week 8221 Derwent Publications Ltd., London, GB; Class A11, AN 82-42503E XP002110800 & JP 57 063302 A (MOTOSATO Y), 16 avril 1982 (1982-04-16)
- DATABASE WPI Section Ch, Week 9125 Derwent Publications Ltd., London, GB; Class B04, AN 91-180992 XP002110801 & JP 03 108661 A (KURITA WATER IND LTD), 8 mai 1991 (1991-05-08)

## Description

La présente invention concerne des compositions comprenant des microparticules poreuses et un agent de suspension, tous deux biocompatibles tant au niveau cellulaire que tissulaire ou général, utilisables pour une implantation dans un tissu pour, en particulier, augmenter le volume de ce tissu (« *soft tissue augmentation* »), en vue notamment de corriger de manière durable un déficit d'aspect ou de fonctionnalité de ce tissu ou organe.

L'invention s'adresse tout particulièrement à la chirurgie plastique et esthétique, à la chirurgie reconstructrice, à la chirurgie urologique.

L'utilisation d'implants pour la correction ou l'atténuation de lésions d'origine organique ou traumatique a connu une accélération rapide et continue depuis le début des années 80. Les matériaux de référence restent le collagène et les dérivés de silicone. D'autres polymères naturels, tels que par exemple l'acide hyaluronique, ou synthétiques prennent une importance croissante.

L'utilisation de dérivés d'origine animale rencontre de plus en plus de résistance, d'abord d'ordre psychologique, ensuite parce que la persistance de leur effet est limitée dans le temps.

De plus, il est connu que les composés d'origine animale, tels que le collagène bovin, sont à l'origine de réactions allergiques dans environ 3 % des cas. La fréquence et la gravité de ces réactions se trouvent aggravées par la nécessité de procéder à des injections répétées nécessaires au maintien d'un résultat satisfaisant. Les dérivés de silicone et d'huile de silicone, bien qu'interdits dans certains pays, restent parmi les plus utilisés pour obtenir un effet durable. Leur utilisation est cependant souvent associée à des problèmes locaux (siliconomes) ou de migration à des distances parfois considérables du site de leur implantation.

Une autre technique connue de comblement consiste à injecter des microsphères de matériaux divers (plastique, verre, céramique, ...) dans les tissus. Ces particules sont en général d'une taille supérieure à la limite d'absorption (phagocytose) par les monocytes, qui vont constituer une pellicule fibreuse à leur surface afin de les isoler des tissus environnants. De telles particules sont décrites par exemple dans le brevet US 5,344,452.

Le résultat de ces injections est généralement durable, mais il est connu que les particules peuvent migrer à distance de leur site d'implantation avec, comme complication, un risque d'obstruction des structures microvasculaires, pouvant entraîner une ischémie ou embolie.

On a maintenant trouvé qu'en utilisant des microparticules ayant une porosité contrôlée et un agent de suspension, par exemple, sous forme de gel, on pouvait obtenir une pénétration par des fibrilles protéiques et/ou glycaniques des tissus implantés permettant un ancrage matriciel micro-fibrillaire limitant significativement les risques de migration à distance du site d'implantation.

L'invention a donc pour objet une composition comprenant :
- des microparticules poreuses dont le diamètre des pores exclut la pénétration d'éléments figurés ayant un poids moléculaire supérieur à 1000 kilodaltons et permet la pénétration de macromolécules microfibrillaires solubles, et
- un agent de suspension biocompatible utilisable en chirurgie plastique, esthétique, reconstructrice et urologique.

La composante solide de la composition selon l'invention est constituée de microparticules poreuses biocompatibles.

Les microparticules utilisées dans la composition selon l'invention auront de préférence une forme sphérique ou ovoïde, d'un diamètre supérieur à environ 10 µm, en particulier compris entre 30 et 100 µm, de préférence entre 30 et 60 µm.

Lorsque les microparticules ne sont pas sphériques, on entend par « diamètre » le plus grand diamètre de la surface ayant la plus petite section transversale.

Les particules de taille inférieure à 10 µm seraient facilement entraînées, activement ou passivement, à distance de leur site d'implantation.

Inversement, l'utilisation de particules d'une taille supérieure à environ 100 µm risquerait de donner aux tissus une rugosité perceptible visuellement ou au toucher dans le cas d'une implantation superficielle. De plus, le produit serait plus difficilement injecté au travers d'une aiguille de petit diamètre, 30G par exemple, nécessaire à une procédure atraumatique.

Une caractéristique avantageuse des microparticules utilisables selon l'invention est qu'elles présentent une structure imparfaitement rigide, permettant une légère déformation en cas de compression. Par «structure imparfaitement rigide », on entend une structure telle que sous l'effet d'une compression axiale d'une valeur maximale compatible avec la résistance du tissu vivant environnant (inférieure ou égale à environ 3 kg/cm²), les particules ne se briseront pas et leur taux de déformation selon cet axe sera compris entre 0 % et 40 %. Cette déformation limitera la tendance au déplacement des particules, phénomène connu dans le cas de particules rigides sous l'appellation « effet graine de melon » (« melon pip effect »), qui est d'autant plus accentué que la surface des microparticules est lisse et lubrifiée.

Les microparticules utilisables selon l'invention, qui peuvent constituer l'essentiel de la composition, présentent une structure poreuse. Le diamètre des pores est déterminé de façon à exclure la pénétration des particules par des éléments figurés de taille supérieure à 1 000 kilodaltons. On recherchera une porosité telle qu'elle favorisera la pénétration de macromolécules de structure naturellement présentes dans les tissus environnant l'implant. La pénétration s'effectue au niveau des macromolécules microfibrillaires solubles. Ces macromolécules, en particulier d'élastine, de collagène ou de glycosaminoglycanes (GAGs), par leur nature microfibrillaire, forment ainsi un réseau finement intriqué qui relie les microparticules qu'elles pénètrent.

En revanche, le diamètre des pores exclut la pénétration des microfilaments ou des filaments macromoléculaires.

Ceci présente l'avantage de contribuer à la formation d'un réseau tridimensionnel assurant l'ancrage des microparticules et, par voie de conséquence, de l'ensemble de l'implant dans la matrice extracellulaire sans provoquer la constitution d'une capsule fibreuse comme ce serait le cas si les microparticules étaient pénétrées par des éléments de taille plus importante ou a fortiori par des fibroblastes, cet ancrage produisant ainsi des résultats anatomiques esthétiques et fonctionnels sensiblement plus satisfaisants.

En particulier, les microparticules ont des pores dont le diamètre exclut la pénétration d'éléments figurés ayant un poids moléculaire compris entre 0,5 kilodalton et 1000 kilodaltons, de préférence entre 1 kilodalton et 1000 kilodaltons.

Le système d'ancrage ainsi obtenu forme une structure souple et élastique dont la cohérence s'accentue dans le temps au fur et à mesure qu'un nombre croissant de liens microfibrillaires en assure la réticulation. La solidité et la consistance des structures ainsi obtenues ressemblent à celles de la matrice extracellulaire, assurant un confort et un aspect optimal.

Cette consistance souple et déformable des éléments comme de l'ensemble, assure un risque minimum de traumatisme, générateur de lésions et d'infection, des tissus.

De préférence, les microparticules sont présentes dans la composition à raison d'environ 0,1 % à 75 % en poids, de préférence de 10 % à 40 %, les pourcentages étant exprimés par rapport au poids total de la composition.

Le matériau constituant les microparticules sera cyto- et histocompatible, et plus généralement biocompatible au sens de la Norme ISO EN 10993. Il sera de préférence un polymère, dont les monomères de départ ne présentent pas de caractère de toxicité incompatible avec l'utilisation envisagée.

On utilisera par exemple un polymère choisi parmi les polyamides, les polyesters ; le polypropylène; le polyéthylène, de préférence « haute densité » ; les dérivés du polyéthylène tels que le polytétrafluoroéthylène, le polyéthylène téréphtalate ; les polyacrylates ; les polyacrylamides; les méthacrylamides ; les polysulfones; les polyvinyles, notamment la polyvinylpyrrolidone (PVP), le divinylbenzène; les polysaccharides éventuellement réticulés; les polylactides et polyglycolides ; les polystyrènes ; les méthylstyrènes ; le dextran ou l'agarose réticulé.

Selon l'invention, on utilisera de préférence un polymère vinylique hydrophile et riche en radicaux hydroxyle (OH), dont la composition atomique est exclusivement constituée de carbone, d'oxygène et d'hydrogène, et dont la polymérisation est totale. Un tel polymère est d'utilisation courante dans la fabrication d'implants et comme constituant de dispositifs médicaux implantables.

Il est également d'utilisation courante au contact de substances chimiques ou biotechnologiques à visée thérapeutique.

Un autre avantage de ce polymère est la disponibilité commerciale de microparticules présentant les caractéristiques techniques et les garanties d'innocuité compatibles avec l'utilisation prévue de l'objet de l'invention.

Les microparticules sont préparées par des procédés usuels décrits dans la littérature, notamment par polymérisation en bloc ou en émulsion.

Dans le cas de la polymérisation en bloc, la solution aqueuse contenant les divers monomères et l'initiateur est soumise à une polymérisation en phase homogène. Le bloc de gel aqueux obtenu est ensuite fractionné en grains, par exemple par passage à travers les mailles d'un tamis.

La polymérisation en émulsion peut fournir directement le gel aqueux sous forme de microparticules de taille déterminée. Elle peut être effectuée par exemple en versant la solution aqueuse contenant les divers monomères dans une phase liquide organique, non miscible à l'eau, maintenue en agitation et contenant éventuellement un agent émulsifiant, puis en introduisant un initiateur de polymérisation.

De tels procédés, qui sont bien connus de l'homme du métier, sont notamment décrits dans la demande EP 040 124.

Une autre technique usuelle dans le domaine est le séchage à contre-courant d'air chaud et sec (« spray drying ») d'un nébulisat de polymère.
Un élément important du bon fonctionnement de l'invention est la nature et les caractéristiques de l'agent de suspension dans lequel les microparticules peuvent être en suspension ou qui peut constituer l'essentiel, voire la totalité, de la composition. Cet agent de suspension peut être un liquide ou un gel.

Cet agent devra permettre de maintenir les particules en suspension dans les conditions normales de conservation et d'utilisation de l'ensemble. A une température et à un pH voisins des conditions de la physiologie, c'est-à-dire un pH compris entre environ 6 et 8 et à une température comprise entre + 25°C et + 40°C, dans des conditions d'isotonicité, ledit agent aura de préférence une densité comprise entre environ 0,85 et 1,35 et une viscosité apparente caractérisée par µo inférieur ou égal à 300 Pa.s et µ_{∞} supérieur ou égal à 10 Pa.s (mesurée selon le modèle rhéologique de Herschel-Buckley).

La consistance de l'agent de suspension sera adaptée au mode d'implantation choisi, par exemple permettant une injection intradermique ou sous cutanée.

La consistance du gel devra être aussi proche que possible de celle du tissu vivant dans lequel il est prévu qu'il soit implanté. Cette consistance variera par exemple entre celle d'un tissu conjonctif pour le moins ferme et celle du cartilage pour le plus ferme. Un façonnage préalable à une implantation chirurgicale plus invasive pourra être réalisé par moulage seul ou complété (tranché, poli, râpé, scié, ...) par utilisation des instruments chirurgicaux habituels.

En principe, tout type de liquide ou de gel biocompatible, peu ou pas résorbable, compatible avec la nature des microparticules peut convenir. Dans le cas d'un gel, celui-ci peut éventuellement être partiellement ou totalement réticulé.

Dans ce cas, la porosité dudit agent de suspension, évaluée par l'espace moyen entre les noeuds de réticulation sera telle qu'elle exclut la pénétration d'éléments figurés ayant un poids moléculaire supérieur à 1 000 kilodaltons.

Dans cet aspect avantageux, la porosité de l'agent de suspension permet la pénétration de macromolécules microfibrillaires présentes dans le milieu comme mentionné plus haut pour les microparticules, tout en excluant les fibrilles ou microfilaments de taille supérieure.

Une caractéristique particulièrement avantageuse de la présente invention consiste dans l'utilisation d'un agent de suspension, de préférence un gel, dont la charge électrique sera globalement positive.

En effet, les macromolécules microfibrillaires assurant une liaison physique entre les microsphères portent des charges négatives. Dans un milieu globalement neutre, la diffusion de ces molécules serait seulement passive, et donc lente et limitée.

Les charges positives présentes au sein de l'agent de suspension, de préférence un gel, auront au contraire un effet attractif pour ces macromolécules, assurant une diffusion active plus rapide et favorisant des concentrations élevées dans l'ensemble de la masse de l'agent de suspension.

Le rapprochement entre les macromolécules microfibrillaires et les microparticules sera ainsi favorisé. Une charge globale négative de l'agent de suspension aurait bien entendu un effet opposé.

L'agent de suspension peut également contenir une ou plusieurs substances pharmacologiquement actives, en particulier un agent analgésique ou anti-inflammatoire.

Le contrôle d'une charge électrique différentielle entre l'implant et le milieu extérieur peut être exploité pour contrôler le taux de diffusion centrifuge ou centripète de substances vers ou en provenance de l'implant, en particulier dans le cas de la délivrance de substances phannacologiquement actives.

De plus, l'utilisation d'un agent de suspension, de préférence un gel, portant une charge électrique globalement positive présente l'avantage d'éviter les calcifications au niveau du site d'implantation.

Selon l'invention, on peut utiliser comme agent de suspension des solutions ou gels constitués ou contenant une solution de polymères d'origine naturelle, tels que par exemple les celluloses, les celluloses modifiées et leur dérivés, comme par exemple la carboxyméthylcellulose, ou encore les polysaccharides cationiques d'origine naturelle, biotechnologique ou synthétique, comme par exemple la chitine, le chitosane et leurs dérivés tels que les dérivés carboxyméthyl-, carboxyéthyl-, N-acyl-, N-carboxyalkyl-, N-carboxyacyl, O-carboxyalkyl, hydroxyalkyl et leurs sels.

Dans un aspect avantageux, on utilisera une solution aqueuse desdits polymères.

Une solution aqueuse de chitosane à 3 % et réticulée, par exemple par un aldéhyde, convient parfaitement à l'application de l'invention à un implant intradermique.

Toutefois, des molécules d'origine naturelle telles que le chitosane sont incompatibles avec des méthodes de stérilisation par rayonnement gamma, ce qui en limite l'utilisation à des dispositifs préparés aseptiquement plutôt que stériles.

Une forme avantageuse de l'invention consiste en l'utilisation d'un gel synthétique, par exemple un polymère d'acrylamide substitué ou non, de vinylpyrrolidone, d'acrylate d'hydroxyalkyle ou un copolymère d'acrylamide substitué ou non substitué et d'une autre molécule portant une charge électrique positive tel qu'un monomère cationique ammonium quaternaire tel que, par exemple, des monomères de type diallyldiméthylammonium, (2-(méthacryloylamino)-propyl) triméthylammonium, (2-(méthacryloyloxy)éthyl)triméthylammonium ou (2-acryloyloxy)éthyl)triméthylammonium disponibles commercialement. La préparation de tels polymères ou copolymères par des techniques usuelles de polymérisation est décrite dans la littérature. Le composé préféré dans le cadre de la présente invention est un gel faiblement réticulé de poly(acrylamide-*co*-diallyldiméthylammonium).

Ce composé dont la biocompatibilité a été montrée conformément aux spécifications de la norme ISO EN 10993 présente l'avantage d'une grande modularité, en fonction de sa concentration et de son taux de réticulation.

Il est en outre parfaitement stérilisable par rayonnement gamma à des doses stérilisatrices égales ou supérieures à 25 kgy.

Il peut facilement être obtenu avec des tailles de maille reproductibles et homogènes, selon des techniques bien connues de l'homme du métier.

La diffusion active des macromolécules microfibrillaires à travers la matrice de ce gel est donc parfaitement assurée de façon également reproductible et fiable, tout en empêchant la diffusion de cellules entières, limitant ainsi au minimum des éventuelles réactions inflammatoires de type dit « à corps étranger ».

Un autre objet de la présente invention est donc de fournir un implant biocompatible, facilement injectable, procurant une augmentation tissulaire durable, et dont les risques de migration à distance du site d'implantation sont limités au minimum.

Selon un de ses aspects, l'invention concerne donc également un implant hétérologue histocompatible comprenant une composition telle que décrite ci-dessus.

L'invention a également pour objet l'utilisation de ladite composition pour la fabrication d'un implant hétérologue histocompatible, ledit implant étant en particulier utilisable en chirurgie plastique, esthétique, reconstructrice et urologique.

Ladite composition peut selon un aspect préféré de l'invention, être utilisée pour fabriquer une prothèse mammaire injectable. Les prothèses mammaires injectables contenant ladite composition représentent un aspect ultérieur de l'invention.

En résumé, la présente invention permet le développement et l'utilisation de matériaux implantables assurant un ancrage durable et atraumatique dans la matrice extracellulaire en particulier.

L'invention est illustrée par les exemples ci-après :

### EXEMPLE 1

Gel injectable par voie intradermique pour la correction durable de rides et de dépressions cicatricielles, du galbe des lèvres, etc...
. microparticules d'acétate de polyvinyle (HW-55F, Sigma Aldrich Fine Chemicals réf 8-07457) 200 mg
. acrylamide 35 mg
. bis acrylamide 1,75 mg
. diallyldiméthylammonium (DADMA) 2 mg
. eau ppi qsp 1 ml.

Des corrections superficielles fines, y compris de lésions de type « vergéture », seront effectuées avec une préparation de l'Exemple 1 ou mieux de l'Exemple 2 ci-dessous.

Ces mêmes compositions sont aussi particulièrement adaptées aux techniques de resurfaçage parfois nécessaires après un traitement par lipoaspiration et pour lesquelles l'injection de graisse autologue est un palliatif imparfait.

### EXEMPLE 2

Gel injectable comme à l'exemple 1, mais destiné à une correction réversible dans le temps
. microparticules d'acétate de polyvinyle (HW-55F, Sigma Aldrich Fine Chemicals réf 8-07457) 200 mg
. chitosane stabilisé 20 mg
. tampon PB qsp 1 ml.

La composition du tampon PB est la suivante :
- Chlorure de magnésium (MgCl₂, 4,5 H₂O) 0,1763 g
- Dihydrogénophosphate de sodium (NaH₂PO₄) 0,0358 g
- Disodium hydrogénophosphate (Na₂HPO₄, 12H₂O) 0,2758 g
- Chlorure de sodium (NaCl) 8,7660 g
- Eau ppi : qsp 1 000 ml

La formule de l'exemple 2 permet une correction durable par apport des microparticules non-résorbables; toutefois, le chitosane est, lui, résorbable et représente environ 50 % du volume initial de l'implant ; le volume résiduel sera donc environ la moitié du volume initial.

Une variante ne contenant pas de microparticules permettra une correction transitoire dont la durée sera limitée à environ 1 an, et devra être renouvelée. Elle sera particulièrement indiquée dans le cas de sites évolutifs, un amaigrissement par exemple pouvant rendre un implant non-résorbable visible et disgracieux. Elle constitue le complément idéal des implants définitifs, ceux-ci ne devant pas apporter une correction totale et en aucun cas une sur-correction.

### EXEMPLE 3

Une variante de l'exemple 1 comportant une plus forte concentration, en particulier de l'agent réticulant, sera particulièrement adaptée au traitement de l'incontinence urinaire, toujours par voie injectable intratissulaire.
. microparticules d'acétate de polyvinyle (HW-55F, Sigma Aldrich Fine Chemicals réf 8-07457) 300 mg
. acrylamide 40 mg
. bis acrylamide 2,5 mg
. DADMA 3 mg
. eau ppi qsp 1 ml.

### EXEMPLE 4

Kit pour correction peu invasive d'hypoplasie mammaire.

Afin de respecter des consistances et des sensations au toucher proches de la normale, autorisant en particulier la continuation de l'autocontrôle par palpation, et de ne pas interférer sensiblement avec les méthodes connues d'imagerie médicale éventuellement prescrites par la suite, les compositions et techniques suivantes sont préférées :
- pour injection au contact du plan musculaire supérieur :
   . microparticules d'acétate de polyvinyle (HW-55F, Sigma Aldrich Fine Chemicals réf 8-07457) 30 g
   . acrylamide 4 g
   . bis acrylamide 200 mg
   . DADMA 200 mg
   . eau ppi qsp 100 ml.
- pour injection au plan sous-glandulaire et superficiel :
   . microparticules d'acétate de polyvinyle (HW-55F, Sigma Aldrich Fine Chemicals réf 8-07457) 15 g
   . acrylamide 3 g
   . bis acrylamide 120 mg
   . DADMA 150 mg
   . eau ppi qsp 100 ml.

## Revendications

1. Composition comprenant :
- des microparticules poreuses dont le diamètre des pores exclut la pénétration d'éléments figurés ayant un poids moléculaire supérieur à 1000 kilodaltons et permet la pénétration de macromolécules microfibrillaires solubles, et
- un agent de suspension biocompatible utilisable en chirurgie plastique, esthétique, reconstructrice et urologique.

2. Composition selon la revendication 1, **caractérisée en ce que** les microparticules sont constituées d'un polymère biocompatible.

3. Composition selon l'une des revendications 1 ou 2, **caractérisée en ce que** les microparticules ont une forme sphérique ou ovoïde de diamètre supérieur à environ 10 µm, de préférence compris entre 30 µm et 100 µm, en particulier entre 30 et 60 µm.

4. Composition selon l'une quelconque des revendications 1 à 3, **caractérisée en ce que** les microparticules ont des pores dont le diamètre exclut la pénétration d'éléments figurés ayant un poids moléculaire compris entre 0,5 kilodalton et 1000 kilodaltons, de préférence entre 1 kilodalton et 1000 kilodaltons.

5. Composition selon l'une quelconque des revendications 1 à 4, **caractérisée en ce que** les microparticules sont présentes dans la composition à raison d'environ 0,1% à 75% en poids, de préférence d'environ 10% à 40%.

6. Composition selon l'une quelconque des revendications 1 à 5, **caractérisée en ce que** les microparticules sont constituées d'un polymère vinylique hydrophile riche en radicaux hydroxyles.

7. Composition selon l'une quelconque des revendications 1 à 5, **caractérisée en ce que** les microparticules sont constituées d'un polymère choisi parmi les polyamides, les polyesters ; le polypropylène ; le polyéthylène,; les dérivés du polyéthylène; les polyacrylates; les polyacrylamides ; les méthacrylamides ; les polysulfones ; les polyvinyles, notamment la polyvinylpyrrolidone, le divinylbenzène ; les polysaccharides éventuellement réticulés ; les polylactides et polyglycolides ; les polystyrènes ; les méthylstyrènes ; le dextran ou l'agarose réticulé.

8. Composition selon l'une quelconque des revendications 1 à 7, **caractérisée en ce que** l'agent de suspension est un liquide ou un gel constitué d'un polymère biocompatible.

9. Composition selon la revendication 8, **caractérisée en ce que** l'agent de suspension est un gel partiellement ou totalement réticulé dont la porosité exclut la pénétration d'éléments figurés ayant un poids moléculaire supérieur à 1000 kilodaltons.

10. Composition selon la revendication 8 ou 9, **caractérisée en ce que** l'agent de suspension a une charge électrique globalement positive.

11. Composition selon l'une des revendications 8 à 10, **caractérisée en ce que** l'agent de suspension est un liquide ou un gel constitué ou contenant une solution de polymères d'origine naturelle choisi parmi les celluloses, les celluloses modifiées et leur dérivés, les polysaccharides cationiques d'origine naturelle, biotechnologique ou synthétique, tels que la chitine, le chitosane, leurs dérivés et leurs sels.

12. Composition selon l'une quelconque des revendications 8 à 10, **caractérisée en ce que** l'agent de suspension est un liquide ou un gel choisi parmi les polymères d'acrylamide substitué ou non, de vinylpyrrolidone, d'acrylate d'hydroxyalkyle ou les copolymères d'acrylamide substitué ou non substitué et d'une autre molécule portant une charge électrique positive telle qu'un monomère cationique ammonium quaternaire.

13. Composition selon la revendication 12, **caractérisée en ce que** l'agent de suspension est un gel de poly(acrylamide-co-diallyldiméthylammonium) faiblement réticulé.

14. Composition selon l'une quelconque des revendications 1 à 13, **caractérisée en ce que** l'agent de suspension contient une ou plusieurs substances pharmacologiquement actives.

15. Utilisation d'une composition selon l'une quelconque des revendications 1 à 14 pour la fabrication d'un implant hétérologue histocompatible.

16. Utilisation selon la revendication 15 pour la fabrication d'un implant hétérologue solide histocompatible utilisable en chirurgie plastique, esthétique, reconstructrice ou urologique.

17. Utilisation selon la revendication 16 pour la fabrication d'une prothèse mammaire injectable.

18. Implant hétérologue histocompatible comprenant une composition selon l'une quelconque des revendications 1 à 14.

19. Prothèse mammaire injectable comprenant une composition selon l'une quelconque des revendications 1 à 14.

## Claims

1. Composition comprising:
- porous microparticles having pores whose diameter prevents formed elements having a molecular weight above 1000 kilodaltons from penetrating and allows soluble microfibril-forming macromolecules to penetrate, and
- a biocompatible suspending agent which can be used in plastic, reconstructive or urological surgery.

2. Composition according to claim 1, **characterized in that** the microparticles consist of a biocompatible polymer.

3. Composition according to either of claims 1 or 2, **characterized in that** the microparticles have a spherical or ovoid form whose diameter is greater than about 10 µm, lies preferably between 30 µm and 100µm, and in particular between 30 µm and 60 µm.

4. Composition according to anyone of claims 1 to 3, **characterized in that** the microparticles have pores whose diameter prevents the penetration of formed elements having a molecular weight lying between 0,5 kilodalton and 1000 kilodaltons, preferably between 1 kilodalton and 1000 kilodaltons.

5. Composition according to anyone of claims 1 to 4, **characterized in that** the microparticles constitute about 0,1% to 75% by weight of the composition, preferably between about 10% to 40%.

6. Composition according to anyone of claims 1 to 5, **characterized in that** the microparticles consist of a hydrophilic vinyl polymer rich in hydroxy groups.

7. Composition according to anyone of claims 1 to 5, **characterized in that** the microparticles consist of a polymer selected from the group consisting of polyamides, polyesters, polypropylene, polyethylene, polyethylene derivatives, polyacrylates, polyacrylamides, methacrylamides, polysulfones, polyvinyls, especially polyvinylpyrrolidone, divinylbenzene, polysaccharides, optionally cross-linked; polylactides and polyglycolides, polystyrenes, methylstyrenes, dextran and cross-linked agarose.

8. Composition according to anyone of claims 1 to 7, **characterized in that** the suspending agent is a liquid or gel consisting of a biocompatible polymer.

9. Composition according to claim 8, **characterized in that** the suspending agent is a partially or totally cross-linked gel whose porosity prevents the penetration of formed elements having a molecular weight above 1000 kilodaltons.

10. Composition according to claim 8 or 9, **characterized in that** the suspending agent has an overall positive electric charge.

11. Composition according to anyone of claims 8 to 10, **characterized in that** the suspending agent is a liquid or a gel consisting of or containing a solution of naturally-occurring polymers selected from the group consisting of celluloses, modified celluloses and their derivatives, naturally-occurring, biotechnological or synthetic polycationic polysaccharides, such as chitin, chitosan, their derivatives and salts.

12. Composition according to anyone of claims 8 to 10, **characterized in that** the suspending agent is a liquid or a gel selected from the group consisting of polymers of substituted or unsubstituted acrylamide, of vinylpyrrolidone, of hydroxyalkyl acrylate or copolymers of substituted or unsubstituted acrylamide and another molecule carrying a positive electric charge such as a quaternary ammonium cationic monomer.

13. Composition according to claim 12, **characterized in that** the suspending agent is a gel of slightly cross-linked poly(acrylamide-*co*-diallyldimethylammonium).

14. Composition according to anyone of claims 1 to 13, **characterized in that** the suspending agent contains one or more pharmacologically active substances.

15. Use of a composition according to anyone of claims 1 to 14 for the manufacture of a histocompatible heterologous implant.

16. Use according to claim 15 for the manufacture of a histocompatible heterologous solid implant usable in plastic, reconstructive or urological surgery.

17. Use according to claim 16 for the manufacture of an injectable mammary prosthesis.

18. Histocompatible heterologous implant comprising a composition according to any of claims 1 to 14.

19. Injectable mammary prosthesis comprising a composition according to any of claims 1 to 14.

## Patentansprüche

1. Zusammensetzung umfassend:
- poröse Mikroteilchen, deren Porendurchmesser die Penetration von Formteilchen mit einem Molekulargewicht von über 1000 Kilodalton ausschließt und die Penetration von löslichen mikrofibrillären Makromolekülen gestattet, und
- ein in der plastischen, ästhetischen, rekonstruktiven und urologischen Chirurgie einsetzbares biokompatibles Suspendiermittel.

2. Zusammensetzung nach Anspruch 1, **dadurch gekennzeichnet, dass** die Mikroteilchen aus einem biokompatiblen Polymer bestehen.

3. Zusammensetzung nach einem der Ansprüche 1 oder 2, **dadurch gekennzeichnet, dass** die Mikroteilchen kugelförmige oder ovale Gestalt mit einem Durchmesser von über etwa 10 µm, vorzugsweise zwischen 30 µm und 100 µm, insbesondere zwischen 30 und 60 µm, haben.

4. Zusammensetzung nach einem der Ansprüche 1 bis 3, **dadurch gekennzeichnet, dass** die Mikroteilchen Poren aufweisen, deren Durchmesser die Penetration von Formteilchen mit einem Molekulargewicht zwischen 0,5 Kilodalton und 1000 Kilodalton, vorzugsweise zwischen 1 Kilodalton und 1000 Kilodalton ausschließt.

5. zusammensetzung nach einem der Ansprüche 1 bis 4, **dadurch gekennzeichnet, dass** die Mikroteilchen in einer Menge von etwa 0,1 Gew.% bis 75 Gew.%, vorzugsweise etwa 10 % bis 40 %, in der Zusammensetzung anwesend sind.

6. Zusammensetzung nach einem der Ansprüche 1 bis 5, **dadurch gekennzeichnet, dass** die Mikroteilchen aus einem hydrophilen Vinylpolymer bestehen, das reich an Hydroxylgruppen ist.

7. Zusammensetzung nach einem der Ansprüche 1 bis 5, **dadurch gekennzeichnet, dass** die Mikroteilchen aus einem Polymer bestehen, ausgewählt aus Polyamiden, Polyestern; Polypropylen; Polyethylen; Polyethylenderivaten; Polyacrylaten; Polyacrylamiden; Methacrylamiden; Polysulfonen; Polyvinylen, insbesondere Polyvinylpyrrolidon, Divinylbenzol; gegebenenfalls vernetzten Polysacchariden; Polylactiden und Polyglycoliden; Polystyrolen; Methylstyrolen; Dextran oder vernetzter Agarose.

8. Zusammensetzung nach einem der Ansprüche 1 bis 7, **dadurch gekennzeichnet, dass** das Suspendiermittel eine Flüssigkeit oder ein Gel, bestehend aus einem biokompatiblen Polymer, ist.

9. Zusammensetzung nach Anspruch 8, **dadurch gekennzeichnet, dass** das Suspendiermittel ein teilweise oder zur Gänze vernetztes Gel ist, dessen Porosität die Penetration von Formteilchen mit einem Molekulargewicht von über 1000 Kilodalton ausschließt.

10. Zusammensetzung nach Anspruch 8 oder 9, **dadurch gekennzeichnet, dass** das Suspendiermittel eine insgesamt positive elektrische Ladung hat.

11. Zusammensetzung nach einem der Ansprüche 8 bis 10, **dadurch gekennzeichnet, dass** das Suspendiermittel eine Flüssigkeit oder ein Gel ist, die bzw. das durch eine Polymerlösung natürlichen Ursprungs, ausgewählt aus Cellulosen, modifizierten Cellulosen und Derivaten davon, kationischen Polysacchariden natürlichen, biotechnischen oder synthetischen Ursprungs, wie Chitin, Chitosan, Derivaten und Salzen davon, gebildet ist oder eine solche enthält.

12. Zusammensetzung nach einem der Ansprüche 8 bis 10, **dadurch gekennzeichnet, dass** das Suspendiermittel eine Flüssigkeit oder ein Gel ist, ausgewählt aus gegebenenfalls substituierten Acrylamidpolymeren, Vinylpyrrolidonpolymeren, Hydroxyalkylacrylatpolymeren oder gegebenenfalls substituierten Acrylamidcopolymeren und einem weiteren Molekül, das eine positive elektrische Ladung aufweist, wie einem kationischen quaternären Ammoniummonomer.

13. Zusammensetzung nach Anspruch 12, **dadurch gekennzeichnet, dass** das Suspendiermittel ein schwach vernetztes Poly(acrylamidco-diallyldimethylammonium)-Gel ist.

14. Zusammensetzung nach einem der Ansprüche 1 bis 13, **dadurch gekennzeichnet, dass** das Suspendiermittel eine oder mehrere pharmakologisch aktive Substanzen enthält.

15. Verwendung einer Zusammensetzung nach einem der Ansprüche 1 bis 14 zur Herstellung eines histokompatiblen heterologen Implantats.

16. Verwendung nach Anspruch 15 zur Herstellung eines histokompatiblen festen heterologen Implantats, das in der plastischen, ästhetischen, rekonstruktiven und urologischen Chirurgie einsetzbar ist.

17. Verwendung nach Anspruch 16 zur Herstellung einer injizierbaren Mammaprothese.

18. Histokompatibles heterologes Implantat umfassend eine Zusammensetzung nach einem der Ansprüche 1 bis 14.

19. Injizierbare Mammaprothese umfassend eine Zusammensetzung nach einem der Ansprüche 1 bis 14.
